**Europäisches Patentamt**

**European Patent Office**

**Office européen des brevets**

(11) Veröffentlichungsnummer: **0 205 032**
**A2**

## (12) EUROPÄISCHE PATENTANMELDUNG

(21) Anmeldenummer: **86107162.9**

(22) Anmeldetag: **27.05.86**

(51) Int. Cl.⁴: **C 12 N 5/00**
**C 12 N 15/00**
**//C12P21/00**

(30) Priorität: **11.06.85 DE 3520844**

(43) Veröffentlichungstag der Anmeldung:
**17.12.86 Patentblatt 86/51**

(84) Benannte Vertragsstaaten:
**AT BE CH DE FR GB IT LI LU NL SE**

(71) Anmelder: **BEHRINGWERKE Aktiengesellschaft**
**Postfach 1140**
**D-3550 Marburg 1(DE)**

(72) Erfinder: **Parwaresch Reza, Prof. Dr.**
**Düsternbrooker Weg 45**
**D-2300 Kiel(DE)**

(72) Erfinder: **Radzun, Jochen, Dr.**
**Lornsenstrasse 34**
**D-2300 Kiel(DE)**

(72) Erfinder: **Kreipe, Hans, Dr.**
**Wilhelminenstrasse 13**
**D-2300 Kiel(DE)**

(74) Vertreter: **Meyer-Dulheuer, Karl-Hermann, Dr. et al,**
**HOECHST Aktiengesellschaft Zentrale Patentabteilung**
**Postfach 80 03 20**
**D-6230 Frankfurt/Main 80(DE)**

(54) **Thymidin-Kinase(EC 2.7.1.21)-Mangelmutanten einer humanen monocytischen Zellinie.**

(57) Es wird ein Verfahren zur Herstellung mutierter Zellen der Zellinie U-937 beschrieben, worin Zellen dieser Zellinie mit einem zur Mutierung tierischer Zellen bekannten chemischen Agenz und dann mit 5'-Bromdesoxyuridin behandelt werden, wobei Zellen erhalten werden, in denen Thymidin-Kinase exprimiert wird.

Diese können als Fusionspartner von M_ nozyten verwendet werden.

Thymidin-Kinase(EC 2.7.1.21)-Mangelmutanten einer humanen
monocytischen Zellinie

---

Die Erfindung betrifft eine Mangelmutante einer permanenten humanen Zellinie, die zur Immortalisierung anderer
Zellen verwendet werden kann.

Die Technik der Immortalisierung von Zellen durch Fusion
mit Zellen permanenter Zellinien ist seit langem bekannt. Solche Hybridzellen oder Hybridoma wurden beispielsweise durch die Fusion von Blutzellen mit Tumorzellen erzielt. In dem klassischen Weg, wie er von Köhler und Milstein (Nature 256, 495-497, 1975) beschrieben
wurde, wurden als permanent wachsende Tumorzellen Myelomzellen der Maus mit B-Lymphozyten der Maus fusioniert. Die aus derartigen Fusionen resultierenden
Hybridzellen sind in der Lage, Stoffe zu sekretieren,
die z. B. aus Kulturüberstand derartiger Hybridzellkulturen einfach und in großen Mengen isoliert werden können. Im Falle der Fusion von B-Lymphozyten mit Myelomzellen produzieren die Hybridzellen eine homogene Antikörperpopulation (monoklonale Antikörper), wobei durch
geeignete Selektionsverfahren solche Hybridzellklone
isoliert und weitergezüchtet werden können, die monoklonale Antikörper einer gewünschten Spezifität produzieren.

Welche Stoffe eine Hybridzelle zu produzieren in der
Lage ist, hängt grundsätzlich von den zur Fusion verwendeten Fusionszellpartnern ab. Werden als Fusionspartner
B-Lymphozyten der Maus mit Myelomzellen verschmolzen, so sind die von einer derartigen Hybridzelle produzierten Stoffe monoklonale Antikörper murinen Ursprungs, während bei Verwendung von humanen B-Lymphozyten als Fusionspartner von Myelomzellen die daraus resultierenden Hybridzellen Antikörper mit allen Charakteristika von menschlichen Immunglobulinen produzieren, unabhängig davon, ob die verwendete Myelomzelle menschlichen oder murinen Ursprungs ist oder sogar selbst eine Xenohybridzelle (Mensch/Maus) darstellt.

In analoger Weise wie es vielfach gelungen ist, B-Lymphozyten mit permanent wachsenden Zellen (ausgewählte Myelomlinien) zu fusionieren, so ist es ebenfalls gelungen, T-Lymphozyten mit geeigneten Fusionspartnern (J.Exp.Med. (1981) 154, 1827-1837) zu verschmelzen. Die von derartigen Hybridomazellen produzierten Stoffe sind, der Natur von T-Zellen entsprechend, Faktoren wie sie von T-Lymphozyten produziert werden können. Derartige Faktoren können z. B. Stoffe wie Interleukine, Interferone, Lymphokine und andere direkt oder indirekt immunregulatorisch wirkende Stoffe sein. Vergleichbar zur B-Lymphozytenfusion können solche Hybride ebenfalls als Produktionsquelle für diese Faktoren benutzt werden, wobei durch geeignete Selektions- und Testverfahren solche Hybridzellen kloniert und weitergezüchtet werden können, die ausschließlich, oder in besonders großer Menge, die gewünschten Stoffe produzieren.

Ebenso wie für die Fusion von B-Lymphozyten mit Myelomzellen haben sich für T-Lymphozyten bisher nur wenige permanente Zellinien als geeignete Fusionspartner erwiesen. Als geeignete Fusionspartner sind solche permanent wachsende Zellinien anzusehen, die in der Lage sind, mit der entsprechenden Partnerzelle zu fusionieren und nach Fusion als Hybridzelle die gewünschten Stoffe zu sekretieren und sowohl von der Partnerzelle als auch von den aus der Fusion resultierenden Hybridzellen separierbar sind. Prinzipiell kann zur Separation von Hybridzellen von den entsprechenden Fusionspartnern jegliches Unterscheidungskriterium (z. B. unterschiedliche Marker, wie sie auch Oberflächenantigene darstellen) ausgenutzt werden. Als besonders geeignetes Selektionsverfahren hat sich jedoch ein Prinzip herausgestellt, bei dem veränderte Wachstumseigenschaften der Hybridzelle gegenüber den Fusionspartnern ausgenützt werden. Zu diesem Zweck kann man als Zelle, die ein permanentes Wachstum gewährleisten soll und in ihren Wachstumseigenschaften verändert ist, beispielsweise eine Mutante einer permanenten Zellinie verwenden, die einen bestimmten Enzymeffekt hat, indem sie beispielsweise im Vergleich zur Mutterzelle nicht in der Lage ist, Thymidin-Kinase (TK; EC 2.7.1.21) zu synthetisieren (TK-Mangelmutante). Solche enzymdefizienten Mutanten permanenter Zellinien sind nicht in der Lage, in geeigneten selektiven Hypoxanthin-Aminopterin-Thymidin-Medien (HAT-Medien) zu überleben. Erst ein Hybridom aus einer derartigen enzymdefizienten Mutante und einer Zelle ohne derartigen Defekt, wie es z. B. eine Blutzelle sein kann, ist in der Lage, in einem derartigen Selektivmedium zu überleben. Verglichen mit anderen bekannten Verfahren stellt ein derartiges Selektionsverfahren zur Isolierung von Hybridzellen von Zellen permanent wachsender Zellinien ein außerordentlich effektives Verfahren dar. Voraussetzung zur Verwendung dieses Verfahrens ist jedoch die Verfügbarkeit einer fusionsfähigen permanent wachsenden Zellinie mit geeigneter Enzymdefizienz.

Die vorliegende Erfindung beschreibt die Herstellung einer fusionsfähigen Zellinie, die als Mutante der Zellinie U-937 (Int. J. Cancer (1976) 17, 565-577) nicht in der Lage ist, Thymidin-Kinase zu synthetisieren und daher in einem Hypoxanthin-Aminopterin-Thymidin enthaltenden Medium (HAT-Medium) nicht wachsen kann und abstirbt. Diese TK-Mutante der Zellinie U-937 kann zu einer Fusion mit Monozyten verwendet werden.

Monozyten sekretieren verschiedene Stoffe, von denen es wünschenswert wäre, sie in größerer Menge herstellen zu können wie Interleukine oder Lymphokine und TNF (tumor necrotizing factor). Zu diesem Zweck wären permanent wachsende Monozyten-Hybride geeignet.

Während die Kenntnisse über B-Lymphozyten verglichen mit denen über andere immunkompetente Zellen sehr groß ist, ist die Erforschung der Eigenschaften von T-Lymphozyten und Monozyten in Nagetieren und in Menschen durch die folgenden Gegebenheiten erschwert:
Obwohl in T-Lymphozyten und Monozyten gewisse funktionelle Aktivitäten ausgelöst werden können, verlangt diese Stimulierung Maßnahmen, die weitere nicht spezifische Effekte auslösen können. Weiterhin ist die Reproduzierbarkeit der Stimulierung gering und es wird nur eine begrenzte Menge sekretierter Produkte erhalten. Auch kann die Zellpopulation, die stimuliert wird, nicht exakt definiert werden, da lediglich eine kleine Zahl von Zellen mit einer begrenzten Wachstumsrate verfügbar ist. Die Anwesenheit selbst einer kleinen Zahl anderer Zelltypen kann nicht vernachlässigt werden angesichts der Tatsache, daß selbst ein kleiner Anteil verunreinigender Zellen erstaunliche biologische Effekte bewirken kann.

Im Gegensatz zu B-Zellhybridzellen ist für Monozytenhybride kein einheitliches Testsystem wie der Immunglobulin-Nachweis möglich. Auf unbekannte Faktoren kann bisher nur in aufwendigen Funktionstestsystemen getestet werden.

Es bestand daher ein Bedürfnis an einer permanenten Zellinie, die es erlaubt, durch Fusion mit Monozyten permanent wachsende Monozytenhybride herzustellen, die diese Schwierigkeiten in der Erforschung von Funktionen von Monozyten überwindet.

Es ist uns überraschenderweise gelungen, durch Behandeln von Zellen der Zellinie U-937, die allgemein zugänglich ist, beispielsweise von verschiedenen Zellbanken, mit Ethylmethan-Sulfonat (EMS) und Behandeln dieser Zellen mit steigenden Konzentrationen von 5´-Bromdesoxyuridin (5´-BU) und Abtrennen der jeweils überlebenden Zellen mutierte Zellen zu erhalten, die Thymidin-Kinase (TK; EC 2.7.1.21) nicht synthetisieren (also eine Thymidin-Kinase-Mangel-Mutante) und in einem Hypoxanthin, Aminopterin und Thymidin enthaltenden Medium (HAT-Medium) nicht wachsen können und absterben.

Gegenstand der Erfindung ist daher eine Mangel-Mutante der permanenten humanen Monozyten/Makrophagenzellinie U-937, dadurch gekennzeichnet, daß die Zellen dieser Mutante Thymidin-Kinase nicht synthetisieren und in einem Hypoxanthin, Aminopterin und Thymidin enthaltenden Medium (HAT-Medium) absterben.

Zellen dieser Mutante sind geeignet als Fusionspartner von Monozyten. Auf diese Weise hergestellte Hybride sind geeignet, von Monozyten sekretierte Stoffe wie Interleukine oder TNF in Zellkultur zu gewinnen. Die beschriebene Mutante von U-937 kann als Zwischenprodukt zur Gewinnung geeigneter Hybride angesehen werden.

Zur Gewinnung dieser Mutante werden Zellen der Zellinie U-937 in einem Medium wie RPMI 1640 unter Zugabe von Antibiotika und gegebenenfalls fötalem Kälberserum wachsen gelassen. Um ein logarithmisches Wachstum zu gewährleisten, sollte die Konzentration der Zellen $5 \times 10^5$ pro ml

- 6 -

nicht übersteigen. Das Medium wurde etwa zweimal pro Woche gewechselt.

Zur Mutation der Zellen wurden sie etwa 12 Stunden lang in einem Medium inkubiert, das etwa 100 µg/ml Ethylmethan-Sulfonat enthielt. Danach wurden die Zellen etwa 3 Tage lang in einem normalen Wachstumsmedium vermehrt.

Zur Selektion mutierter Zellen wurde dann einer Suspension der Zellen in normalem Kulturmedium, beispielsweise RPMI-Medium, 3 µg/ml 5´-BU zugesetzt und 3 Tage einwirken gelassen. Danach wurden die überlebenden Zellen durch Zugabe von soviel 12-O-tetradecanoylphorbol-13-acetat (TPA), daß eine Konzentration von $1 \times 10^{-9}$ mol/l erreicht wurde, und 72-stündiges Stehen zum Anhaften an die Glasoberfläche gebracht und auf diese Weise abgetrennt. Die Selektion mit 5´-BU wurde dann mit einer Konzentration von 6, 9 und dann 18 µg/ml wiederholt und jeweils die überlebenden Zellen auf die beschriebene Weise abgetrennt. Die Zellen die 18 µg/ml 5´-BU überlebten, wurden einem "semi-cloning step" unterworfen, indem sie in einer Zelldichte von $1 \times 10^4$ Zellen pro ml der Einwirkung von Konzentrationen an 5´-BU von 30, 42 und 50 µg/ml ausgesetzt wurden. Dann wurden jeweils 20 Zellen/ml kloniert und die einzelnen Klone auf ihr Wachstumsverhalten in HAT-Medium ($1 \times 10^{-4}$ mol/l Hypoxanthin, $4 \times 10^{-7}$ mol/l Aminopterin und $4 \times 10^{-5}$ mol/l Thymidin) geprüft, bevor sie weiter vermehrt und analysiert wurden.

Die Zellklone wurden autoradiograpisch oder szintillationsspektrometrisch mit tritium-markiertem Thymidin auf die Abwesenheit von Thymidin-Kinase-Aktivität geprüft. Normale U-937-Zellen bzw. Kulturmedium dienten als positive bzw. negative Kontrollen.

Etwa die Hälfte der U-937-Zellen überlebten die 48-stündige Behandlung mit Ethylmethan-Sulfonat und die folgenden 3 Tage in normalem Kulturmedium wie mit dem Trypanblau-Ausschlußtest gezeigt werden konnte. In den Selektionsschritten mit steigenden Konzentrationen des toxischen Thymidin-Analogon starben jeweils 70-90 % der Zellen ab. Die Wachstumsgeschwindigkeit der selektionierten Zellen nahm auf etwa 50 % des Wertes der ursprünglichen U-937-Zellen ab. Im Klonierungsschritt am Ende konnte in etwa jedem zweiten Ansatz Wachstum beobachtet werden.

Sechs Klone wurden ausgewählt aufgrund ihrer höheren Wachstumsgeschwindigkeit in normalem Medium und ihrer Empfindlichkeit gegen HAT-Medium, worin sie innerhalb von 48 Stunden völlig abstarben. Während die unbehandelten Ausgangszellen von U-937 in der Autoradiographie eine dichte Anhäufung von Schwärzungspunkten in der Kerngegend zeigten, ergaben sich bei den TK-negativen Klonen bis zu höchstens 10 Schwärzungspunkten pro Zelle, die wohl wegen geringer mitochondrienassoziierter TK-Aktivität über das Zytoplasma verteilt waren. Die 6 ausgewählten Klone zeigten eine Thymidinaufnahme von höchstens 7,8 % der ursprünglichen Zellen. Die entsprechenden Einzelwerte waren nicht signifikant verändert, wenn sie nach einem halben Jahr nochmals bestimmt wurden.

Eine morphologische Betrachtung der ausgewählten Klone zeigte keine Unterschiede. Dasselbe trifft zu für die enzmyzytochemischen Charakteristika. Die Zellen zeigten gerundete Kerne und einen dünnen, leicht basophilen Zytoplasmasaum. Während eine Prüfung auf Naphthol AS-D-Chloracetat-Esterase-Aktivität stets negativ war, ergab die Prüfung auf saure Esterase und saure Phosphatase starke diffuse oder granuläre Reaktionsmuster.

Eine Bestimmung der sauren Esterase und der sauren Phosphatase in einer isoelektrischen Fokussierung ergab, daß die beschriebenen Mutanten das typische Isoenzymmuster normaler humaner Monozyten zeigten. Dementsprechend wurden 5 saure Esterase Isoenzyme zwischen pH 5,7 und 6,25 gefunden sowie 11 Isoenzyme der sauren Phosphatase im pH-Bereich von 4-6,3.

Jeweils mehr als 80 % der Zellen der beschriebenen 6 Klone von Mangelmutanten reagierten mit den monoklonalen Antikörpern, die unter den Bezeichnungen OKM, Ki-M3 und Ki-M7 bekannt sind.

Ein Vergleich des Phenotyps der Mangelmutanten mit dem normaler U-937-Zellen zeigte keine wesentlichen Unterschiede.

Patentansprüche:

1. Mangelmutante der permanten humanen Monozyten/Makrophagen-Zellinie U-937, dadurch gekennzeichnet, daß die
   Zellen dieser Mutante Thymidin-Kinase (EC 2.7.1.21)
   nicht synthetisieren und in einem Hypoxanthin, Aminopterin und Thymidin enthaltenden Medium (HAT-Medium)
   absterben.

2. Verwendung von Zellen der Mutante nach Anspruch 1 als
   Fusionspartner von Monozyten.

3. Verfahren zur Herstellung einer Mutante nach Anspruch
   1, dadurch gekennzeichnet, daß Zellen der permanenten
   humanen Monozyten/Makrophagen-Zellinie U-937 mit
   einem zur Mutierung tierischer Zellen bekannten chemischen Agenz, vorzugsweise Ethylmethan-Sulfonat, und
   dann mit Konzentrationen von 5´-Bromdesoxyuridin, die
   stufenweise von 3 bis mindestens 50 µg/ml steigen,
   behandelt werden, wobei jeweils die überlebenden
   Zellen abgetrennt und in die folgende Stufe eingesetzt werden und die schließlich erhaltenen Zellen,
   in denen praktisch keine Thymidin-Kinase exprimiert
   wird, vermehrt werden.